# EUROPEAN PATENT SPECIFICATION

(11) **EP 0 491 114 B1**
(45) Date of publication and mention of the grant of the patent: **01.03.1995**
(21) Application number: 91112217.4
(22) Date of filing: 22.07.1991
(51) Int. Cl.: C12P 21/00, C12N 1/38, C12N 1/06, C12N 5/00

(54) **A process for preparing new non-covalent polysaccharide-protein associations having pharmacological activity**
Verfahren zur Herstellung neuer nichtkovalenten Polysaccharid-Protein-Zusammensetzungen mit pharmakologischen Eigenschaften
Procédé pour la préparation de nouveaux complexes non-covalents protéine-polysaccharide doués d'activité pharmacologique

(30) Priority: 18.12.1990 ES 9003173
(43) Date of publication of application: 24.06.1992
(73) Proprietor: LABORATORIOS ANDROMACO S.A., 28028 Madrid (ES); CORLAY, S.A., E-28028 Madrid (ES)
(72) Inventor: Guerrero Gomez-Pamo, Antonio, E-28007 Madrid (ES); De Lacy Boville, Rafael, E-28042 Madrid (ES); Matji Tuduri, José Antonio, E-28043 Madrid (ES); Pivel Ranieri, Juan Pablo, E-28014 Madrid (ES)
(74) Representative: Garcia Cabrerizo, Francisco

(56) References cited:
- WO-A-89/04599
- FR-A- 2 379 604
- FR-A- 2 582 672
- GB-A- 2 025 768

## Description

### Field of the invention

The invention relates to a process for preparing non-covalent polysaccharide-protein associations (hereinafter Ps-Pr NCA) and to their application in human and animal medicine in the different pharmaceutical forms thereof.

### Background of the invention

The scientific and technical literature (Chihara et al., Cancer Res, 30, 2776, 1970; Chihara et al., Nature, 222, 687, 1969; Teukagoshi, Cancer Chemotherapy, 1, 251, 1974 and Kamei et al., Cancer Chemotherapy, 4, 166, 1977), describes the preparation and pharmacological properties of polysaccharides (Ps) isolated from cultures of certain fungi, yeasts and bacteria. Particularly important among other properties of interest are the anti-tumor, anti-viral, immunomodulating, hypocholesterolemic, hypolipidemic, hypoglucemic, reticulo-endothelial system activator, etc. activities.

The most immediate prior art to the present invention is ES 543.855, wherein there is described a process for preparing a nitrogenated polysaccharide (N-Ps), whereby the fermentation is produced by consecutive inoculation separated in time of two pure cultures of two different genera of bacteria, Zymomonas and Pediococcus. There then follows a lysis-hydrolysis process by chemical and enzymatic action, filtration and preparation of the pharmacologically active N-Ps adsorbate.

The known processes suffer from several drawbacks defined as those derived from the handling of pure microbial cultures, such as the need to use sterile media, pollution, conservation of the genetic integrity of the strains, as well as those derived from the limited biological potential of the single specifically inoculated microorganisms relative to the processing of the substrates. Unfavourable consequences of this are variability in the substrate conversion or in the biosynthesis of the products, which means lack of reproducibility in the yields and/or in the characteristics of the endproduct, which may not be corrected by the subsequent processes of fractioning and purification.

The present invention discloses a process free from the above drawbacks, since the process described is a "mixed culture", in accordance with the definition and having the corresponding advantages proposed by Hesseltine (C.W. Hesseltine -1983- Ann. Rev. Microbiol. 37, 575-601), consisting fundamentally of a spontaneous fermenting process, i.e. without pure inocula, the development of which is based essentially on the pressure of biological selection produced by a special culture medium, unique to the present invention, which causes the same natural microorganisms always to grow in the same time sequence, thereby defining a fermentation standard. And furthermore, this invention affords the advantage that said medium produces a fermentation standard and extraction characteristics such that the use of different precursors is made possible. These, processed always in the same way, lead to their corresponding active end products. The advantages may be summarized, therefore, as:
1. A process has been achieved allowing pure inocula of laboratory strains to be replaced by naturally occurring microorganisms, developed by selection pressure in "mixed culture", with the corresponding elimination of the risk of loss of the process by contamination or modification of the genetic features of the strains used.
2. A process has been achieved which, being a mixed culture of naturally occurring strains, allows different precursors both of the polysaccharide component and of the protein component of the different end products defined as Ps-Pr NCA to be processed. This versatility of the process of the present invention is not possible with processes based on pure inocula, capable of efficaciously modifying only their specific substrates. Thus, different new pharmacologically active Ps-Pr NCA are prepared.
3. Other purification processes of Ps-Pr NCA have been developed, allowing them to be used in other more effective galenic forms.

The Ps-Pr NCA have their own pharmacological properties allowing them to be applied in human and animal medicine.

### Brief summary of the invention

The object of the invention is a process for preparing pharmacologically active non-covalent polysaccharide-protein associations (Ps-Pr NCA) consisting of:
a) preparing a culture medium by addition, under stirring, to a particular volume of deionized water, of metal oxides, divalent metal salts, bacteriostats, elementary sulphur and the precursors generating the protein fraction and the polysaccharide fraction. The suspension, after homogenization, is held without stirring at a temperature ranging from 35° to 45°C, there being produced under these conditions a fermentation characterized by acidulation of the medium, which is maintained until a pH ranging from 3 to 5 is reached.
b) producing a controlled chemical and enzymatic hydrolysis at pH 1.0-2.8 and at a temperature of 35°-45°C by incorporating in the previously filtered fermentation medium a strong inorganic or organic acid (pKa 0.1-3.0), a chaotropic agent and a protease active in acid medium, to obtain as sole macromolecules in solution the components of the Ps-Pr NCA; and
c) purifying the Ps-Pr NCA by removing the low molecular weight contaminants by i) dialysis/exhaustive ultra- filtration processes using appropriate membranes, which supply the pure Ps-Pr NCA in true solution or ii) precipitation by addition over a water miscible organic solvent, in the presence of inorganic calcium salts, in insoluble absorbate form. Both processes provide products which are used for the preparation of the different pharmaceutical forms.

The precursors are empirically selected biological materials carrying the molecules which will produce those forming part of the end product, the Ps-Pr NCA. Said molecules form part of tissue or cell structures from which they are released to be extracted and modified. The protein fraction (Pr) generating precursors comprise a number of naturally occurring products, among which they are selected from one or several seeds, grains or nuts, such as rape seeds, groundnuts, castor beans, sunflower seeds, soya beans, rice, their delipified residual mixtures or meals. The polysaccharide (Ps) fraction generating precursors are dried dead yeasts, one being selected from among the genera Candida, Saccharomyces, Rhodotorula, Sporobolomyces, Hansenula, Pichia, or their dry extracts.

For the purposes of the present invention, any possible polysaccharide fraction precursor pair (hereinafter PR-Ps)/ protein fraction precursor or precursors (hereinafter PR(s)-Pr) may be used indifferently, from among those mentioned above. Each PR-Ps/PR(s)-Pr pair gives a corresponding pharmacologically active Ps-Pr NCA, after a fermenting process defined by the conditions of the medium and subsequent purification.

The object of the present invention is the preparation of pharmacologically active polysaccharide-protein associations of interest in human and animal medicine. To this end, there is described a process characterized by:
- A number of precursors of the protein fraction and of the polysaccharide fraction of the Ps-Pr NCA selected empirically on the basis of the biological activity of the corresponding end product and from which the molecules forming said fractions will be obtained.
- A fermentation/extraction medium characterized by the properties of controlling the fermentation process of the microorganisms provided naturally by their organic components and of extracting and modifying the molecules which will form the Ps-Pr NCA.
- Selective controlled chemical, physical and enzymatic conditions of hydrolysis for the purposes of hydrolyzing all the macromolecular components extracted except those which will form part of the pharmacologically active Ps-Pr NCA.
- Ps-Pr NCA purification processes by way of dialysis/ultrafiltration or the formation of an insoluble adsorbate producing the products which are used for the preparation of the different galenic forms.

The process of the present invention is characterized in that it comprises the following steps:
In a first step, a culture medium is prepared by the addition under stirring to a particular volume of deionized water, of metal oxides, such as manganese dioxide, manganese and cobalt salts, with the anions from hydrochloric or sulphuric acid, known bacteriostats such as camphor, elementary sulphur, and organic acids of the abietic group in their natural form of rosin (70% acid). After homogenization the suspension is held at a temperature ranging from 35° to 45°C.
In a second stage the Ps-Pr NCA precursors are prepared and added to the culture medium.
In a third step the fermentation medium is held at rest under a controlled temperature ranging from 35° to 45°C. On reaching a pH ranging from 3 to 5, the process is held for a further 4 to 13 days, after which this step of the process is considered to be terminated and a fourth step is performed in which the liquid filtrate of the fermentation process is treated with strong acids having a pKa of 0.1-3.0 such as hydrochloric, trichloroacetic, trifluoroacetic, phosphoric or sulphuric acid, to a pH ranging from 1 to 2.8, and chaotropic agents and lysing enzymes active in the pH range of 1-3, which may be selected from among pepsin, papain, chemopapain and bromelin, with incubation for 6 to 72 hours at a temperature ranging from 35° to 45°C. The Ps-Pr NCA is found in solution after the above described treatments.
The fifth step of the process consists of concentrating the solution from the previous step. After filtration to clean the medium, tangential ultrafiltration is performed through membranes having a molecular cut-off of 10,000 to 50,000 daltons. In this way the volume is reduced to a final one ranging from 1/3 to 1/18 of the initial volume.
In the sixth step, the material may be alternatively purified by selective adsorption or by exhaustive dialysis and subsequent evaporation to dryness or drying in the presence of salts:
   Alternative (a): The purification by selective adsorption is effected by the addition of calcium chloride and adjusting the medium pH followed by a change of polarity by addition of a water miscible organic solvent such as ethanol or acetone, thereby separating the Ps-Pr NCA in the form of an insoluble adsorbate.
   Alternative (b): The purification by exhaustive dialysis is effected by diafiltration of the solution from the fifth step against deionized water using membranes having a molecular cut-off of 10,000 to 50,000 daltons. Evaporation to dryness by lyophilization or spray drying produces the soluble form of the end product.
   Alternative (c): Drying in a muffle, in the presence of inorganic stabilizing salts such as calcium suphates and phosphates produces the insoluble form, similar to the adsorbate.
In the seventh step, the products obtained in the previous step are processed to prepare the different galenic forms:
   - Capsules, sachets or tablets are prepared from the insoluble inorganic salt stabilized forms (Alternatives (a) or (c)).
   - Aqueous solutions with or without absorption promotors, suspensions, microemulsions, suppositories, injectables or topical preparations are prepared from the soluble forms (Alternative b).

The products prepared as described in the processes of Examples 1 to 4 show a number of pharmacological properties which are described below and which are the basis of the therapeutic use in human and animal medicine.

### Reduction of the tumor necrosis factor (TNF) levels in serum of mice treated with bacterial endotoxin (LPS)

The product described in Example 1 was administered to Balb/c mice orally at a dose level of 150 mg/kg for 6 consecutive days, prior to intravenous challenge with 25 »g/animal of E. coli endotoxin serotype 055 B5. The result of the treatment with the product described in Example 1 leads to an inhibition of over 50% in the serum TNF levels obtained 90 minutes after the administration of LPS. Similar results were obtained in animals sensitized to the endotoxin with Zymosan or the Calmette-Guerin bacillus. The TNF determinations were made by measuring the cytotoxicity of the serum versus the cell line L929.

### Reduction of the cholesterol level in hypercholesterolemic rats treated with Triton

The i.p. administration of Triton WR1239 dissolved in saline solution at dose levels of 500 mg/kg to Wistar rats induces hypercholesterolemia. When the product described in Example 1 was administered at a dose level of 500 mg/kg incorporated in the Triton solution, there was a reduction of the serum cholesterol levels of over 40% versus a control group. The serum cholesterol was determined 18 hours after i.p. administration of the products.

### Increase of base corticosterone levels in mice

When the product described in Example 1 was administered orally to Balb/c mice at a dose level of 150 mg/kg, there was an increase of over 200% relative to the base corticosterone serum levels, measured by a radio-immunoanalysis 5 hours after product administration.

A similar result was obtained when the product described in Example 2 was administered via rectum at a dose level of 1,600 mg/kg.

### Increase of the haematopoietic activity valued by the number of precursor cells (CFU-S and CFU-GM)

Intravenous administration of the product described in Example 3 to C57B1/6 rats at a dose level of 0.4 mg/kg produced an increase of over 150% in the number of spleen pluripotent cells (CFU-S) and 200% in the number of granulocite-macrophage line precursor spleen cells (CFU-GM), measured 5 days after administration versus a control.

### Increase of the spleen haematopoietic activity valued by the increase in the number of pluripotent cells (CFU-S) after sublethal radiation

Oral administration of the product described in example 4 to C57B1/6 rats at a dose level of 150 mg/kg for six consecutive days after a sublethal radiation of 5.5-6.0 Gy produced an increase of 60% in the number of pluripotent spleen cells (CFU-S) versus the control.

### Increase in survival of immunosuppressed mice infected with Listeria monocytogenes

Oral administration of the product described in Example 4 to Swiss mice at a dose level of 150 mg/kg for 6 days prior to infection produced protection in rats immunodepressed with silica and infected with Listeria monocytogenes. The immunosuppression is induced by treatment with 120 mg/kg i.p. of silica 1 day before provoking infection. The protection is evidenced as an increase in the LD₅₀, which in the treated animals reached a value similar to that of the non-immunosuppressed animals.

### Protective effect in mice subjected to lethal radiation

The product prepared according to Example 3 when administered in a single i.v. dose of 0.4 mg/kg⁻¹ to C57B1/6 mice one day before receiving a lethal radiation dose (7.75 Gy) produced a 100% survival rate valued 15 days thereafter.

The analytical profile of the different products prepared on operating up to step 6 inclusive of the processes described in Examples 1 to 4 is shown in Table I.

**TABLE I**

| | Example 1 | Example 2 | Example 3 | Example 4 |
|---|---|---|---|---|
| Organic matter % (w/w) | 4.1-9.9 | 90-100 | 90-100 | 4.1-9.9 |
| Salts % (w/w) | 91-95 | 0-10 | 0-10 | 91-95 |
| Polysaccharide fraction % (w/w) (1) | 2.8-7.7 | 55-85 | 55-85 | 2.8-7.7 |
| Protein fraction % (w/w) (2) | 1.3-2.2 | 15-45 | 15-45 | 1.3-2.2 |

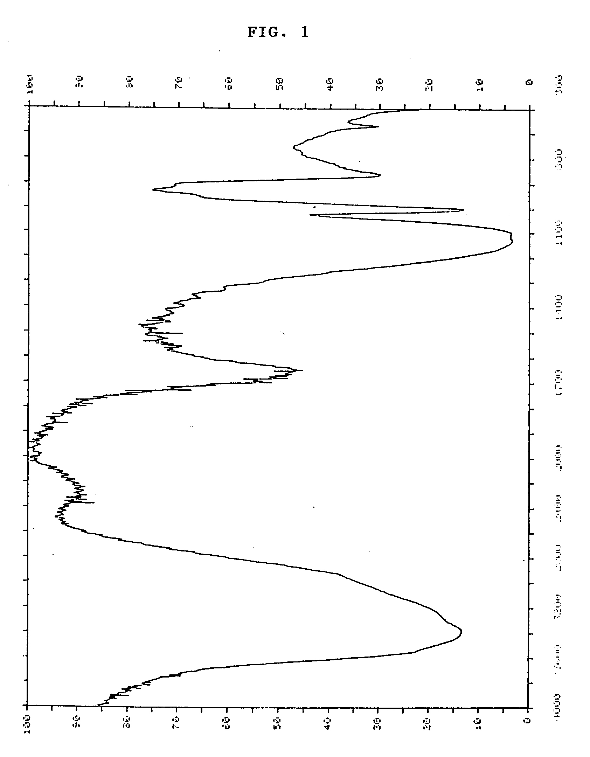

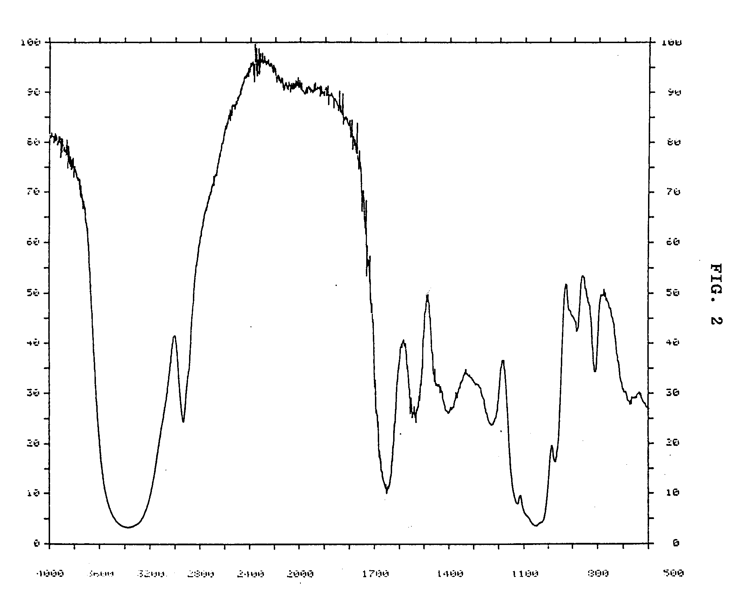

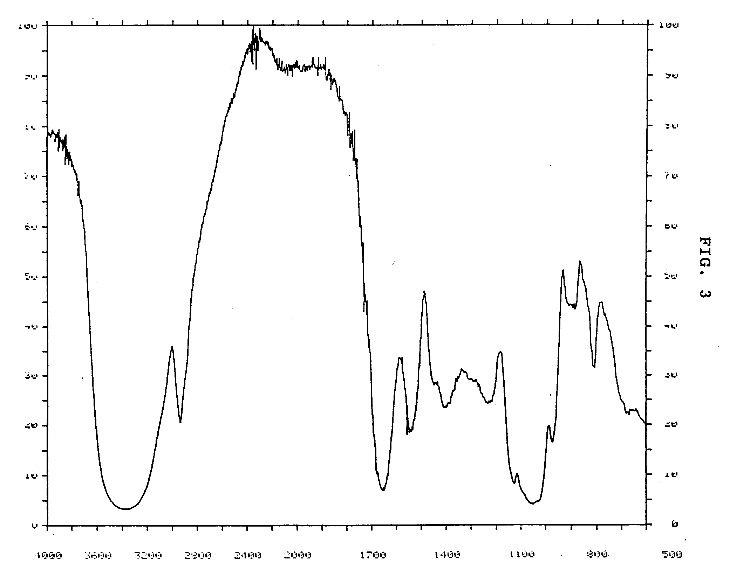

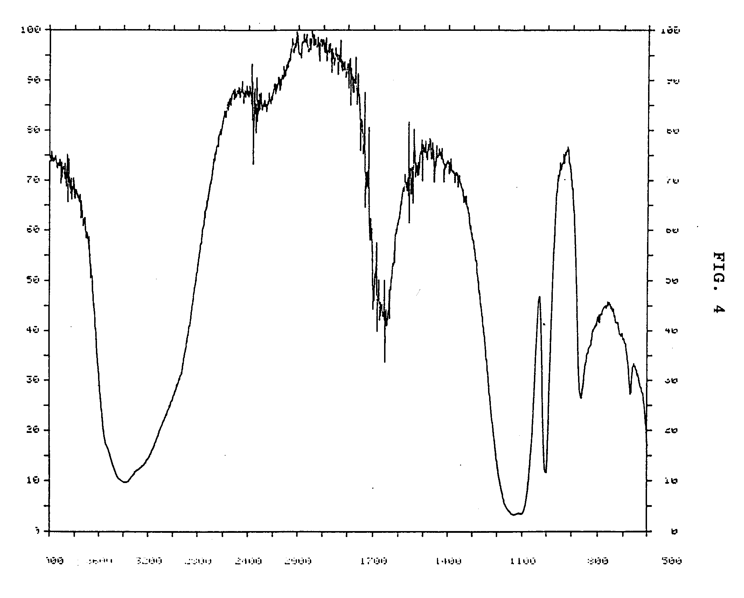

(1): Determined according to the method of Dubois (Dubois A. et al. Anal. Chem. 28, 350, 1956). (2): Determined according to the method of Lowry (Lowry O.H. et al., J. Biol. Chem. 193, 265, 1951).
Polysaccharide fraction having a molecular weight ranging from 10⁴ to 3 x 10⁵, (formed by a heteropolysaccharide (formed by mannose (70-90% w/w), glucose (5-20% w/w) and galactose (0-3% w/w), with 1-6 and/or 1-2, 1-3 or 1-4 bonds). Protein fraction having a molecular weight ranging from 10⁴ to 2 x 10⁵.
The IR spectra (KBr) of the non-covalent Ps-Pr associations prepared according to Examples 1 to 4 are given in Figures 1 to 4 respectively.

The infrared spectra of the non-covalent polysaccharide-protein associations prepared on operating the processes described in Examples 1 to 4 are shown in Figures 1 to 4 respectively. Said spectra were prepared with potasium bromide tablet, with a specimen concentration of 2% in a Perkin-Elmer model 881 infrared spectrophotometer, scanning from 4,000 to 600 cm⁻¹ in 6 minutes with a variable slot, resolution at 1,000 cm⁻¹ and spectral noise of 0.5% T. The spectra are shown electronically corrected by the Savitzky/Golay process and automatically expanded in absorbance.

### Example 1

First step: Preparation of the culture medium.
   From 0.2 to 0.4 g of manganese bioxide, from 5.2 to 7.1 g of manganese sulphate, from 2 to 5 g of rosin, from 2.4 to 4.2 g of cobalt chloride, from 4 to 6 g of camphor and from 4.5 to 6.5 g of sulphur precipitated in a 2 litre reactor were added to 1,000 ml of deionized water. The mixture was held at a temperature ranging from 35° to 45°C.
Second step: Preparation and addition of precursors.
   From 5 to 25 g of soya beans, previously washed and soaked in water, were milled and added to the culture medium prepared in the previous step. Thereafter from 3 to 12 g of dead, dried Candida utilis were added.
Third step: Fermentation - extraction.
   The fermentation mixture was held at rest at a temperature ranging from 35° to 45°C until the medium pH was between 3.0 and 5.0, the process being held for a further 4 to 13 days.
Fourth step: Chemical and enzymatic hydrolysis.
   The reaction medium was filtered in Büchner funnel through a layer of diatomaceous earth and 85% phosphoric acid was added to the filtrate to reach a pH between 1.0 and 2.8. Thereafter from 6 to 12 g of urea and from 0.1 to 0.6 g of pepsin was added. After homogenization, the mixture was held at rest for 6 to 72 hours at 35°-45°C.
Fifth step: Concentration.
   The reaction mixture from the previous step was filtered in a Büchner funnel through a layer of diatomaceous earth and concentrated by ultrafiltration through a polysulfone membrane having a molecular cut-off level of 10,000 to 50,000 daltons. Thus the volume was reduced to approximately 1/5 of the starting volume.
Sixth step, alternative a: Adsorbate.
   From 10 to 25 g of anhydrous calcium chloride were added to the concentrate of the previous step, the pH being adjusted thereafter to 3.5-4.4 by the addition of 10% NaOH solution. This solution was poured over acetone, the final proportion of acetone being adjusted to 25 - 40% (volume/volume). The mixture was allowed to stand for 24 hours, and thereafter the major portion of the supernatant was decanted. The precipitate was filtered in a Büchner funnel, was washed exhaustively with an acetone/water mixture between 25 and 40% (volume/volume) and dried in an oven with air ventilation.
Seventh step: Galenic form.
   The product prepared in the previous step was diluted with a mixture of CaHPO₄.2H₂O/CaSO₄.2H₂O 2/1 until the total sugar concentration measured by the Dubois method was about 1.2-2.0% (w/w).

### Example 2

First step: Preparation of the culture medium.
   The culture medium was identical to the one described in Example 1.
Second step: Preparation and addition of precursors.
   Likewise from 5 to 25 g of soya beans, washed, soaked in water and ground were used. In this case the polysaccharide source was from 3 to 12 g of dead, dried Saccharomyces cerevisiae.
Third step: Fermentation-extraction.
   The fermentation mixture was worked up in the same way as in Example 1.
Fourth step: Chemical and enzymatic hydrolysis.
   The filtration, acidulation and hydrolysis were conducted identically to the method described in Example 1.
Fifth step: Concentration.
   The filtered medium was concentrated by ultrafiltration under the same conditions as described for Example 1. The final volume is 1/5 of the starting volume.
Sixth step, alternative b: Soluble product.
   The concentrate from the previous step was subjected to exhaustive dialysis by concentration cycles by ultrafiltration and addition of deionized water up to de original volume. The dialysis proceeded until the electric conductivity of the permeate had a value similar to that of the deionized water. The solution was then concentrated by ultrafiltration to a volume ranging from 1/6 to 1/10 of the original, and was finally lyophilized.
Seventh step: Galenic form.
   Suppositories containing 0.125% (w/w) in Supocire BP, labrafil type excipient, were prepared from this lyophilized product.

### Example 3

First step: Preparation of the culture medium.
   Identical to the method described in Examples 1 and 2.
Second step: Preparation and addition of precursors.
   A mixture of from 2.9 to 14.6 g of soya beans and from 2.1 to 10.4 g of castor beans, ground after being washed and soaked in water and from 3 to 12 g of dead, dried Candida utilis was used.
Third step: Fermentation-extraction.
   The fermentation mixture was worked up in the same way as in Example 1.
Fourth step: Chemical and enzymatic hydrolysis.
   Identical to that described for Examples 1 and 2.
Fifth step: Concentration.
   Conducted in the same way as described in Example 1, the solution being concentrated 6 times.
Sixth step, alternative b: Soluble product.
   Identical to that described in Example 2.
Seventh step: Galenic form.
   The product prepared in the previous step was dissolved extemporaneously for intravenous injection in saline serum solution.

### Example 4

First to fifth steps:
   In the same way as Example 3, concentrated to 1/6 of the initial volume.
Sixth step, alternative a: Adsorbate.
   The same as for Example 1.
Seventh step: Galenic form.
   As described for Example 1.

## Claims

1. A process for preparing non-covalent polysaccharide-protein (Ps-Pr) associations, wherein:
a) one or several precursors of the protein fraction (Pr) selected from among oleaginous seeds, grains or nuts, and one or several precursors of the polysaccharide fraction (Ps) selected from among yeasts or extracts thereof, are reacted together at a temperature ranging from 35° to 45° C with a selective controlled extraction-fermentation aqueous medium, consisting of cobalt and manganese compounds, elementary sulphur, abietic acid or abietates and a bacteriostatic agent, the mixture being held at rest, with the absence of provoked bacterial inocula;
b) thereafter, the resulting Ps-Pr solution is subjected to a selective controlled chemical and enzymatic hydrolysis at pH 1.0-3.0 and at the same temperature, incorporating an acid having a pKa 0.1-2.0, a chaotropic agent and an enzyme selected from the group formed by pepsin, papain, chemopapain or bromelin; and
c) the Ps-Pr adsorbate is precipitated from the filtered concentrated liquors in the presence of salts, by adding a protic or aprotic water miscible organic solvent to the reaction medium, or by dialysis-ultrafiltration, to isolate the pure Ps-Pr association and prepare the different galenic forms.

2. The process of claim 1, wherein the aqueous extraction-fermentation medium is prepared preferably with the following composition : metal oxides, from among which there are selected manganese dioxide at a concentration of 0.2 to 0.4 g L⁻¹ ;
manganese and cobalt salts with anions from hydrochloric or sulphuric acid, at respective concentrations of 1.9 to 2.6 g L⁻¹ and 4.0 to 6.0 g L⁻¹;
elementary sulphur, from 4.5 to 6.5 g L⁻¹ and organic acids from the group formed by abietic acid in its natural form of rosin, preferably having a 70% acid content at a concentration of 2.0 to 5.0 g L⁻¹.

3. The process of claim 1, wherein from the naturally occurring precursor products generating the protein fraction, there are selected seeds, grains or nuts from the group formed by rape seed, groundnut, castor beans, sunflower seeds, soya beans, rice, the delipidated residues or meals thereof, and from the precursors generating the polysaccharide fraction there are selected the dead dried yeasts or their dry raw extracts from among the genera Candida, Saccharomyces, Rhodotorula, Sporobolomyces, Hansenula, Pichia.

4. The process of claim 1, wherein the amount of precursor material added to the extraction-fermentation medium is selected from among 5.0 to 25.0 g L⁻¹ for the seeds and the equivalent in dry weight for the meals, and 3.0 to 12.0 g L⁻¹ for the precursor material of the polysaccharide fraction.

5. The process of claim 1, wherein the extraction-fermentation medium is held at a temperature of 35° to 45°C in the absence of stirring and at rest, the fermenting process being held for 4 to 13 days, with pH controlled to 3.0-5.0, to obtain a solution containing the molecules forming the non-covalent polysaccharide-protein association.

6. The process of claim 1, wherein the chemical and enzymatic hydrolysis is conducted by the addition to the filtered medium of:
an acid selected from the group formed by hydrochloric, phosphoric, sulphuric, trichloroacetic or trifluoromethanesulphonic acid to pH 1-2.8;
a chaotropic agent selected from urea at a concentration of 6.0 to 12.0 g L⁻¹;
lysing enzymes selected from the group formed by papain, chemopapain, pepsin or bromelin, in an amount of 0.1 to 0.6 g L⁻¹; the incubation proceeding for 6 to 72 hours at a temperature of 35° to 45°C.

7. The process of claim 1, wherein the concentration of the liquids is conducted by membranes having a molecular cut-off ranging from 10,000 to 50,000 daltons.

8. The process of claim 1, wherein the non-covalent polysaccharide-protein association is purified in insoluble adsorbate form, when the acid used in the lysis is phosphoric acid, with calcium chloride being added to a final concentration of from 10.0 to 25.0 g L⁻¹, sodium hydroxide in solution to pH ranging from 3.5 to 4.4 and a water miscible solvent such as ethanol or acetone to a final concentration ranging from 25 to 40% (w/w).

9. The process of claim 1, wherein the non-covalent polysaccharide-protein association is purified by exhaustive dialysis through membranes having molecular cut-off ranging from 10,000 to 50,000 daltons, and thereafter is isolated by evaporation to dryness, lyophilization, spray drying or concentration and drying in the presence of salts such as calcium sulphate and phosphate.

10. Use of the non-covalent polysaccharide-protein associations prepared according to the process of claim 1 in the manufacture of a medicament for restoring immunomodulating and haematopoietic activities in humans and animals.

11. The process of claim 1, wherein the non-covalent polysaccharide-protein associations exhibit immunomodulating and haematopoietic restoring pharmacological activity.

12. The process of claim 1, wherein the Ps-Pr associations of :
Candida - Soya
Candida - Castor
Candida - Soya : Castor
Saccharomyces - Soya
Saccharomyces - Castor
Saccharomyces - Castor : Soya
their adsorbates and mixtures with salts are selected.

## Patentansprüche

1. Verfahren für die Zubereitung von nicht kovalenten Polysaccharid - Protein - Verbindungen (Ps - Pr), worin:
a) ein oder mehrere Vorläufer der Proteinfraktion (Pr), die unter ölhaltigen Samen, Körnern oder Nüssen ausgewählt werden sowie ein oder mehrere Vorläufer der Polysaccharidfraktion (Ps), die unter Hefen oder deren Extrakten ausgewählt werden, bei einer Temperatur von 35º bis 45º C zusammen mit einer selektiv kontrollierten Extraktion - Fermentation wässrigem Mediums umgesetzt werden, die aus Kobalt- und Manganzusammensetzungen, elementarem Schwefel. Abietinsäure oder Abietinen und einem bakteriostatischen Agens besteht, indem man die Mischung in Abwesenheit von hervorgerufener Bakterienokulierung ruhen läßt;
b) anschließend wird die sich daraus ergebende Ps - Pr - Losung bei 1.0 - 3.0 pH und gleicher Temperatur einer selektiv kontrollierten chemischen und enzymatischen Hydrolyse ausgesetzt, indem eine Säure mit 0.1 - 2.0 pKa, ein chaotropisches Agens und ein Enzym beigesetzt werden, das von der Gruppe ausgewählt wird, die von Pepsin, Papain, Chemo-papain oder Bromelin gebildet wird; und
c) die adsorbierte Ps - Pr - Substanz wird von den gefilterten konzentrierten Flüssigkeiten in Abwesenheit von Salzen abgeschieden, indem man dem Reaktionsmedium ein mischbares organisches Lösungsmittel protischen oder aprotischen Wassers beifügt oder durch Dialysen - Ultrafiltrierung, um die reine Ps- Pr - Verbindung zu trennen und um die unterschiedlichen galenischen Formen vorzubereiten.

2. Verfahren nach Anspruch 1, worin das wässrige Mittel der Extraktion - Fermentation vorzugsweise in folgender Zusammensetzung vorbereitet wird: Metalloxyde, unter denen Mangandioxid in einer Konzentration von 0.2 bis 0.4 g L⁻¹ ausgewählt wird;
Mangan- und Kobaltsalze mit Anionen aus Chlorwasserstoff oder Schwefelsäure in Konzentrationen von 1.9 bis 2.6 g L⁻¹ bezw. 4.o bis 6.0 g L⁻¹;
elementarer Schwefel von 4.5 bis 6.5 g L⁻¹ und organische Säuren aus der Gruppe, die aus Abietinsauren und ihrer natürlichen Kolophoniumform gebildet wird, die vorzugsweise einen Säuregehalt von 70 % bei einer Konzentration von 2.0 bis 5,0 g L⁻¹ aufweisen soll.

3. Verfahren nach Anspruch 1, worin es von den natürliceh vorkommenden Vorläuferprodukten, welche die Proteinfraktion erzeugen, ausgewählte Samen, Körner oder Nüsse gibt, die aus der Gruppe stammen, die von Rübsamen, Erdnüssen, Rizinus, Sonnenblumensamen, Soja, Reis, deren Rückstände ohne Lipoide oder Mehl sowie von den Vorläufern gebildet wird, welche die Polysaccharidfraktion erzeugen, indem man die toten getrockneten Hefen oder deren trockene rohe Extrakte unter den Gattungen von Candida, Saccharomyces, Rhodotorula, Sporobolomyces, Hansenula, Pichia auswählt.

4. Verfahren nach Anspruch 1, worin die Menge des Vorläufermaterials, das dem Mittel der Extraktion - Fermentation beigefügt wird, von 5.0 bis 25.0 g L⁻¹ für Samen und die gleichwertige Menge Trockengewicht für Mehl und von 3.0 bis 12.0 g L⁻¹ für das Vorläufermaterial der Polysaccharidfraktion ausgewählt wird.

5. Verfahren nach Anspruch 1, worin das Extraktion - Fermentationsmittel bei einer Temperatur zwischen 35º und 45º C gehalten wird, ohne es umzurühren und indem man es ruhen läßt, wobei dieser Gärungsprozeß von 4 bis 13 Tage mit von 3.0 - 5.0 kontrolliertem pH aufrechterhalten wird, um eine Lösung zu erhalten, die die Moleküle enthält, welche die nicht kovalente Polysaccharid - Protein - Verbindung bildet.

6. Verfahren nach Anspruch 1, worin die chemische und enzymatische Hydrolyse geleitet wird, indem man dem gefilterten Mittel die folgenden Substanzen beifügt:
eine Säure, die aus der Gruppe gewählt wird, die von Chlorwasserstoff, Phosphorsäure, Schwefelsäure, Trichloressigsäure oder Trifluoromethan-Sulfonsäure gebildet wird. zu 1 - 2.8 pH;
ein chaotropisches Agens, das aus Harnstoff in einer Konzentration von 6.0 bis 12.0 g L⁻¹ ausgewählt wird;
Lyso-Enzyme, die aus der Gruppe gewählt werden, die von Papain, Chemo-Papain, Pepsin oder Bromelin gebildet wird und zwar in einer Menge von 0.1 bis 0.6 g L⁻¹, wobei eine Latenzzeit von 6 bis 72 Stunden bei einer Temperatur zwischen 35º und 45º C aufrechterhalten wird.

7. Verfahren nach Anspruch 1, worin die Konzentration der flüssigen Mittel über Membrane kontrolliert wird, deren molekularer Trennpunkt von 10.000 bis 50.000 Daltons reicht.

8. Verfahren nach Anspruch 1, worin die nicht kovalente Polysaccharid - Protein - Verbindung in Form eines unauflöslichen Adsorbats veredelt wird und wozu die in der Lyse benutzte Säure eine Phosphorsäure ist, indem man der Endkonzentration von 10.0 bis 25.0 g L⁻¹ Kalziumchlorid, Natriumhydroxyd in einer Lösung von 3.5 bis 4.4 pH und ein in Wasser mischbares Lösungsmittel wie z. B. Aethanol oder Azeton einer Endkonzentration zwischen 25 und 40 % (W/W) beifügt.

9. Verfahren nach Anspruch 1, worin die nicht kovalente Polysaccharid - Protein - Verbindung durch sorgfältige Dialyse über Membrane mit molekularem Trennpunkt von 10.000 bis 50.000 Daltons veredelt wird und anschließend wird sie abgesondert mittels Verdunsten bis zur Trockenheit, Lyophilisation, Zerstäubungstrocknung oder Konzentration und Trockung in der Anwesenheit von Salzen wie z. B. Kalksulfat und Phosphat.

10. Benutzung nicht kovalenter Polysaccharid - Protein - Verbindungen, die nach dem Verfahren des Anspruches 1 zubereitet wurden, um ein Medikament zur Wiederherstellung der immunomodulatorischen und hämatopoietischen Aktivitäten in Menschen und Tieren herzustellen.

11. Verfahren nach Anspruch 1, worin die nicht kovalenten Polysaccharid - Protein - Verbindungen immunomodulatorische und hämatopoietische Merkmale zur Wiederherstellung pharmakologischer Aktivität aufzeigen,

12. Verfahren nach Anspruch 1, worin die Ps - Pr - Verbindungen von
Candida - Soja
CAndida - Rizinus
Candida - Soya: Rizinus
Saccharomyces - Soja
Saccharomyces - Rizinus
Saccharomyces - Rizinus; Soja
sowie deren Adsorbate und Mischungen mit Salzen ausgewählt werden.

## Revendications

1. Un procéde pour la préparation d'associations non covalentes de polysaccharides-protéines (Ps-Pr), dans lequel,
a) un ou plusieurs précurseurs de la fraction de protéines (Pr), choisis parmi des semences, grains ou noix oléagineux, et un ou plusieurs précurseurs de la fraction de polysaccharides (Ps), choisis parmi les levures et leurs extraits, sont mis à réagir ensemble à une température comprise entre 35º C et 45º C, avec un milieu aqueux d'extraction-fermentation contrôlé de façon sélective, consistant en composés de cobalt et manganèse, soufre élémentaire, acide abiétique ou abiétates, ainsi qu'un agent bactériostatique, le mélange étant maintenu au repos en l'absence d'inoculation bactérienne provoquée.
b) la solution Ps-Pr résultante est ensuite soumise à une hydrolyse chimique et enzymatique sélective contrôlée, avec un pH 1.0 - 3.0, et à la même température, avec incorporation d'un acide de pKa 0,1 - 2.0, un agent caotropique et un enzyme sélectionné du groupe formé par la pepsine, la pepaïne, la chémopapaïne ou la promeline; puis,
c) l'absorbate de Ps-Pr est précipité dans les liqueurs filtrées en présence de sels, en ajoutant au milieu de réaction un dissolvant organique protéique ou aprotéique, miscible dans l'eau, ou au moyen d'un ultrafiltrage par dialyse, dans le but d'isoler l'association Ps-Pr pure et de préparer les différentes formes galéniques.

2. Le procédé de la revendication 1, dans lequel le milieu de fermentation-extraction aqueux se prépare de préférence avec les compositions suivantes: oxydes métalliques, parmi lesquels on sélectionne le dioxyde de manganèse, à un taux de 0,2 à 0.4 g L⁻¹;
sels de manganèse et cobalt avec des anions d'acide chlorhydrique ou sulfurique, à des taux de 1,9 à 2.8 g L⁻¹ et 4.0 à 6.0 g L⁻¹, respectivement;
soufre élémentaire, de 4,5 à 6.5 g L⁻¹, et acides organiques du groupe formé par l'acide abiétique sous sa forme naturelle de colophane, contenant de préférence 70 % d'acide, à un taux de 2.0 à 5.0 g L⁻¹.

3. Le procédé de la revendication 1, dans lequel, parmi les produits précurseurs naturels qui génèrent la fraction de protéine, on sélectionne les semences, grains ou noix du groupe formé par les graines de colza, de cacahouète, de ricin, de tournesol. de soja, le riz, les résidus sans lipides de leurs farines, et, parmi les précurseurs qui génèrent la fraction de polysaccharides, on sélectionne les levures mortes sèches ou leurs extraits crus secs parmi les genres Candida, Saccharomyces, Rhodotorula, Sporobolomyces, Hansenula, Pichia.

4. Le procédé de la revendication 1 dans lequel la quantité de matériel précurseur qui est ajouté au milieu de fermentation-extraction est de 5.0 à 25.0 g L⁻¹, pour les graines et l'équivalent en poids sec pour les farines, et de 3,0 à 12.0 g L⁻¹ pour le matériel précurseur de la fraction de polysaccharide.

5. Le procédé de la revendication 1, dans lequelle moyen de fermentation-extraction est maintenu à une température de 35º à 45º C, en l'absence d'agitation et au repos, le processus de fermentation étant maintenu de 4 à 13 jours, en contrôlant le pH à 3.0 - 5.0, afin d'obtenir une solution qui contienne les molécules qui forment l'association non covalente de protéine-polysaccharide.

6. Le procédé de la revendication 1, dans lequel l'hydrolyse chimique et enzymatique est contrôlée par l'addition au milieu filtré de:
un acide sélectionné dans groupe formé par les acides chlorhydrique, phosphorique, sulfurique, trichloracétique ou trifluorométhanosulfonique, à un pH de 1 - 2.8;
un agent caotropique choisi de l'urée, à un taux de 6.0 à 12,0 g L⁻¹;
enzymes choisis dans le groupe formé par la papaïne. chémopapaïne, pepsine ou broméline, dans une quantité de 0.1 à 0.6 g L⁻¹; l'incubation est effectuée pendant une période de 6 à 72 heures, à une température de 35º à 45º C.

7. Le procédé de la revendication 1, dans lequel le taux des liquides est contrôlé au moyen de membranes dont la gamme de coupe moléculaire se situe entre 10.000 et 50.000 daltons.

8. Le procédé de la revendication 1, dans lequel l'association de protéines polysaccharides non covalentes est purifiée sous forme d'absorbate insoluble, lorsque l'acide utilisé por la lyse est l'acide phosphorique; on ajoute du chlorure de calcius à un taux final de 10.0 à 25.0 g L⁻¹, de l'hydroxyde de sodium en solution à un pH de 3.5 à 4.4 et un dissolvant miscible dans l'eau tel que le méthanol ou l'acétone, jusqu'à un taux final de 25 à 40 % (W/W).

9. Le procédé de la revendication 1, dans lequel l'association de protéines polysaccharides non covalentes est purifiée par dialyse exhaustive à travers une membrane dont la gamme de coupe moléculaire se situe entre 10.000 et 50.000 daltons, puis est isolée par évaporation jusqu'a la sécheresse, par lyophilisation, par séchage, par pulvérisation ou par concentration et séchage en présence de sels, tels que le sulfate et le phosphate de calcium.

10. L'usage des associations de protéines-polysaccharides non covalentes préparées selon le procédé de la revendication 1, pour la préparation d'un médicament destiné à rétablir les activités immunomodulantes et hématopoïétiques, chez l'homme et les animaux.

11. Le procédé de la revendication 1, dans lequel les associations de protéines polysaccharides non covalentes présentent une activité pharmacologique reconstituante, immunomodulante et hématopoïétique.

12. Le procédé de la revendication 1, dans lequel sont sélectionnées les associations Ps-Pr- de:
Candida - Soja
Candida - Ricin
Candida - Soja; Ricin
Saccharomyces - Soja
Saccharomyces - Ricin
Saccharomyces - Ricin: Soja
ainsi que leurs absorbates et leurs mélanges avec des sels.
